# EUROPEAN PATENT SPECIFICATION

(11) **EP 4 129 483 B1**
(45) Date of publication and mention of the grant of the patent: **25.06.2025**
(21) Application number: 21911131.7
(22) Date of filing: 25.01.2021
(51) Int. Cl.: B01L 7/00, C12Q 1/686, B01L 9/00

(54) **PORTABLE RT-PCR DEVICE AND RT-PCR MEASUREMENT METHOD USING SAME**
TRAGBARE RT-PCR-VORRICHTUNG UND RT-PCR-MESSVERFAHREN DAMIT
DISPOSITIF DE RT-PCR TRANSPORTABLE ET PROCÉDÉ DE MESURE DE RT-PCR UTILISANT CELUI-CI

(30) Priority: 22.12.2020 KR 20200181069
(43) Date of publication of application: 08.02.2023
(73) Proprietor: Gene2us Corp., Cheonan-si, Chungcheongnam-do 31151 (KR)
(72) Inventor: RYU, Seong Ho, Cheonan-si Chungcheongnam-do 31163 (KR)
(74) Representative: AWA Sweden AB
(86) International application number: PCT/KR2021/000946
(87) International publication number: WO 2022/139061

(56) References cited:
- KR-A- 20100 008 476
- KR-A- 20120 031 188
- KR-A- 20140 029 142
- KR-A- 20140 029 142
- KR-A- 20140 128 671
- KR-A- 20140 128 671
- KR-B1- 100 773 561
- KR-B1- 101 040 489
- US-A1- 2010 159 582

## Description

### [Technical Field]

The present disclosure relates to a gene amplification (PCR) device and, more particularly, to a portable real-time PCR (RT-PCR) device and an RT-PCR measurement method that uses the portable RT-PCR device.

### [Background Art]

Usually, the DNA amplification techniques have been broadly utilized for research and development and diagnosis in the biological science, genetic engineering, and medicine fields. Particularly, the DNA amplification techniques that use the polymerase chain reaction (PCR) have been widely utilized.

The polymerase chain reaction (PCR) is used when amplifying specific DNA sequences, as required, that are present in a genome.

The polymerase chain reaction is a method of possibly amplifying a DNA region between two primers to a large amount in a test tube. For DNA synthesis, a DNA polymerase needs a primer. From this primer, DNA is synthesized in the direction from 5' to 3'. Using this synthesis, the following cycle is repeated: ① Denaturation to single-stranded DNA, ② annealing of primers, and ③ synthesis of complementary DNA due to a polymerase. Thus, only a target gene region is proliferated in the test tube.

To this end, in the PCR, DNA denaturation is first performed. Double-stranded DNA can be separated by being heated. DNA resulting from the separation serves as a template.

Next, in the PCR, an annealing step is performed. In this step, the primers are bounded to template DNA. An annealing temperature is an important factor in determining the reaction accuracy. When the annealing temperature is set to be too high, the primers too weakly bonds to the template DNA. Thus, an amount of DNA resulting from the amplification is very small. In addition, when the annealing temperature is set to be too low, the primers are non-specifically bounded to the template DNA. Because of this, undesired DNA can be amplified.

Next, in the PCR, an elongation step is performed. In this step, the heat-resistant DNA polymerase creates new DNA from the template DNA.

Real-time polymerase chain reaction (real-time (RT) RCR) is also referred to as quantitative polymerase chain reaction (qPCR). In the case of usual PCR, after the reaction is completed, the quantity of final products can be determined. In contrast, in the case of the RT-PCR, while the reaction proceeds, a process of amplifying a DNA molecule can be quantitatively observed.

When the RT-PCR takes place, a reporter probe bonds to the middle of DNA, but fluorescence still does not appear. In an RT-PCR amplification step, when the forward-direction primer is caused to extend, a Taq DNA polymerase meets the reporter probe. At this point, when the report probe is broken down by a function of enzyme breakdown from 5' to 3' that is retained by the Taq DNA polymerase, a fluorescent label is separated from a fluorescent quenching label. Thus, fluorescent appears.

This process is performed in a fluorometer that measures fluorescence. Therefore, when fluorescence is measured, the extent to which PCR proceeds can be measured. When the sufficient quantity of reporter probes is present, the reporter probe bonds to new DNA that is created time after time. Thus, the more increased an amplification period, the more increased the amount of fluorescence time after time.

In a PCR device in the related art, it is not easy to perform control to maintain desired temperature by performing a method of raising and then lowering temperature in each step. Accordingly, there is a limitation in that the polymerase chain reaction does not smoothly proceed.

In addition, a specimen accommodation container accommodating a specimen needs to be formed in the shape of a tube that extends over a long distance in the upward-downward direction. Moreover, a separate pipette needs to be used in order to accommodate the specimen into the tube. Therefore, there is a problem in that it is difficult to directly preform PCR measurement in an external test environment. KR101040489 discloses a PCR apparatus capable of real time monitoring, wherein the apparatus comprises a plurality of blocks having a PCR tube, a rotor to which the PCR tube is mounted, and a second guide groove for forming a path for moving the rotor, wherein the block is disposed and forms one rotor movement path with the second guide groove, and a sensing means for irradiating light onto a DNA sample accommodated in the PCR tube, wherein the plurality of blocks are arranged in each step of the DNA synthesis process. The apparatus also comprises a cooling block for cooling the PCR tube which is located between the first and second thermostable blocks and which accommodates the DNA sample. KR20140029142 discloses a PCR machine comprising one or more modules including a device unit and a measuring unit located on the device unit; and a supporting pillar penetrating the center of the device unit. The device unit comprises a temperature region formation unit including heating blocks with different temperature regions; and a rotary unit located on the upper or lower sides of the temperature region formation unit by touching the temperature region formation unit, and having chip insertion units located on one of the heating blocks. KR20140128671 discloses a polymerase chain reaction (PCR) apparatus includes a sample unit which for receiving materials used for synthesizing DNA; a temperature controlling unit which is installed near the sample unit, moves toward the sample unit, and controls the temperature of the sample unit be the temperature of each step required for DNA synthesis; and a sensor module which determines the degree of DNA amplification and DNA by irradiating light to the sample unit. US20100159582 discloses a polymerase chain reaction (PCR) device including a chip assembly, a plurality of chambers being provided in said chip assembly adapted to hold samples, heating means wherein said chip assembly being located on said heating means whereby said chip assembly is allowed to operatively rotate on said heating means, a rotary wheel aiding said chip rotation and wherein said heating means comprises of plural temperature zones in a manner that on rotation of said chip means said sample chamber is shifted from one temperature zone to another by means of a rotary-linear motion system.

### [Summary of Invention]

### [Technical Problem]

Accordingly, an object of the present disclosure is to provide a portable RT-PCR device capable of easily performing temperature control and thus smoothly performing a polymerase chain reaction and an RT-PCR measurement method that uses the portable RT-PCR device.

Another object of the present disclosure is to provide a portable RT-PCR device capable of being easily used and an RT-PCR measurement method that uses the portable RT-PCR device.

### [Solution to Problem]

According to an aspect of the present disclosure, there is provided a portable RT-PCR device including: a base unit in which a mounting space is formed; a plurality of lower heating units mounted in the mounting space in the base unit; a lower optical measurement unit mounted in the base unit and arranged in a different position than the plurality of lower heating units and providing measurement light or receiving the measurement light; and a chamber assembly including a plurality of chambers that are seated on the plurality of lower heating units and the lower optical measurement unit, respectively, each chamber being provided in such a manner to be movable from one of the plurality of lower heating units to other one of the plurality of lower heating units or to the lower optical measurement unit, wherein each of the plurality of chambers includes: a chamber body for accommodating a chamber unit in which a specimen-unit accommodation space inside which a specimen unit is accommodated is formed; wherein the chamber body is formed to receive the chamber unit having a chamber unit body in which the specimen-unit accommodation space is formed to accommodate the specimen unit having an aspect ratio, that is, a height-to-width ratio, which is greater than 0 and smaller than 1.; and a cap unit covering the specimen-unit accommodation space in the chamber unit body from above, and wherein the portable RT-PCR device further comprising a cover unit arranged over the base unit and covering the mounting space; a plurality of upper heating units each of which is arranged between each of the plurality of lower heating units and the cover unit and which are selectively brought into contact with upper surfaces, respectively, of the plurality of chambers; and an upper optical measurement unit facing the lower optical measurement unit, receiving the measurement light emitted from the lower optical measurement unit or providing the measurement light toward the lower optical measurement unit; and wherein the plurality of upper heating units and the plurality of lower heating units are formed in such a manner that a distance between each of the plurality of upper heating units and each of the plurality of lower heating units is variable, wherein, in a case where each of the plurality of chambers is arranged between each of the plurality of upper heating units and each of the plurality of lower heating units and is not moved for a maintenance time, the distance between each of the plurality of upper heating units and each of the plurality of lower heating units corresponds to a height of each of the plurality of chambers, and wherein, in a case where the maintenance time expires and where the plurality of chambers are moved toward other upper heating units, respectively, and toward other lower heating units, respectively, the distance between each of the plurality of upper heating units and each of the plurality of lower heating units is set to be greater than the height of each of the plurality of chambers.

In the portable RT-PCR device, the chamber assembly may further include: a chamber movement unit for moving the plurality of chambers, wherein the plurality of chambers may be arranged around a rotational center formed in the base unit in such a manner as to be spaced a preset distance apart, and wherein the chamber movement unit may rotate the plurality of chambers around the rotational center in one direction at the same time.

In the portable RT-PCR device, the chamber movement unit may include: a plurality of connection brackets, first end portions of which are connected to the plurality of chambers, respectively; and a rotation shaft to which second end portions of the plurality of connection brackets are connected and which is provided in a manner that is rotatable about the rotational center, wherein the rotation of the chamber movement unit may be stopped for a maintenance time, and the chamber movement unit is rotated for a movement time, and wherein the maintenance time may be set to be longer than the movement time.

In the portable RT-PCR device, a first guide unit for guiding the rotation of each of the plurality of chambers may be formed to be positioned between one of the plurality of lower heating units and other one of the plurality of lower heating units or the lower optical measurement unit, the first guide unit may be formed in such a manner as to have a curvature corresponding to a curvature radius of an imaginary circle that is formed when the plurality of chambers are rotated, and a guide groove or a guide protrusion that is engaged with the first guide unit may be formed in or on a lower portion of each of the plurality of chambers.

In the portable RT-PCR device, a second guide unit that is connected to the first guide unit, has the same curvature radius as the first guide unit, and is selectively engaged with the guide groove in each of the plurality of chambers or the guide protrusion thereon may be formed to be positioned on upper surfaces of the lower heating unit and the lower optical measurement unit.

In the portable RT-PCR device, the lower heating unit may include: a first lower heating unit that operates at a first temperature; a second lower heating unit that operates at a second temperature; and a third lower heating unit that operates at a third temperature, the first lower heating unit and the third lower heating unit may be symmetrical about the rotational center, and the second lower heating unit and the lower optical measurement unit may be symmetrical about the rotational center.

In the portable RT-PCR device, the first temperature may be set to be higher than the third temperature, and the third temperature may be set to be higher than the second temperature, and the first lower heating unit, the second lower heating unit, and the third lower heating unit may be kept at their respective set temperatures.

In the portable RT-PCR device, the plurality of upper heating units and the plurality of lower heating units are formed in such a manner that a distance between each of the plurality of upper heating units and each of the plurality of lower heating units is variable, in a case where each of the plurality of chambers is arranged between each of the plurality of upper heating units and each of the plurality of lower heating units and is not moved for a maintenance time, the distance between each of the plurality of upper heating units and each of the plurality of lower heating units correspond to a height of each of the plurality of chambers, and, in a case where the maintenance time expires and where the plurality of chambers are moved toward other upper heating units, respectively, and toward other lower heating units, respectively, the distance between each of the plurality of upper heating units and each of the plurality of lower heating units are set to be greater than the height of each of the plurality of chambers.

In the portable RT-PCR device, the plurality of lower heating units each may be formed in the shape of a plate, lower surfaces of the plurality of chambers may be brought into full contact with the plurality of lower heating units, respectively, a chamber-unit insertion space into which the chamber unit is to be inserted may be formed in the chamber body of each of the plurality of chambers, and the chamber-unit insertion space may be formed in such a manner that a width thereof corresponds to a width of each of the plurality of chamber units.

In the portable RT-PCR device, a measurement solution may be accommodated in the specimen-unit accommodation space in each of the plurality of chamber units, and the specimen-unit accommodation space may be formed in such a manner that the aspect ratio thereof is greater than 0 and smaller than 1, and the specimen-unit accommodation space may be formed in such a manner that a volume thereof is 20 µl to 100 µl.

In the portable RT-PCR device, the specimen unit may be formed with a membrane structure formed of a porous material.

According to another aspect of the present disclosure, there is provided an RT-PCR measurement method that uses the portable RT-PCR device mentioned above, the method including: a specimen-unit input step of accommodating a plurality of chamber units, in each of which the specimen unit is accommodated, into the plurality of chambers, respectively; a heating and measurement operation starting step of performing a heating or measurement operation on the specimen unit in a state where the specimen unit is input; a chamber-assembly one-step movement step of moving the plurality of chambers by one step in a case where a maintenance time in the heating and measurement operation starting step is longer than a preset reference maintenance time; and a measurement result notification step of providing notification of a result of measurement in a case where a measurement cycle for the plurality of chambers is set to be longer than a preset reference cycle.

The RT-PCR measurement method may further include: a distance-between-heating-units increasing step of increasing a distance between each of the plurality of upper heating units that are arranged to face the plurality of lower heating units, respectively, and each of the plurality of lower heating units, before the chamber-assembly one-step movement step is performed, in a case where the maintenance time in the heating and measurement operation starting step is longer than a preset reference maintenance time; and a distance-between-heating-units decreasing step of decreasing the distance between each of the plurality of upper heating units and each of the plurality of lower heating units after the chamber-assembly one-step movement step is performed, wherein in the distance-between-heating-units increasing step, the plurality of upper heating units and the plurality of lower heating units are formed in such a manner that the distance between each of the plurality of upper heating units and each of the plurality of lower heating units is greater than a height of each of the plurality of chambers, and wherein in the distance-between-heating-units decreasing step, the distance between each of the plurality of upper heating units and each of the plurality of lower heating units corresponds to the height of each of the plurality of chambers.

In the RT-PCR measurement method, one measurement cycle may be defined as four steps by which each of the plurality of chambers is moved.

In the RT-PCR measurement method, in the chamber-assembly one-step movement step, the plurality of chambers may be rotated by a preset angle about a rotational center formed in the base unit, the plurality of lower heating units may include a first lower heating unit that operates at a first temperature, a second lower heating unit that operates at a second temperature, and a third lower heating unit that operates at a third temperature, the plurality of upper heating units that face the plurality of lower heating units, respectively, may include a first upper heating unit that faces the first lower heating unit and operates at the first temperature, a second upper heating unit that faces the second lower heating unit and operates at the second temperature, and a third upper heating unit that faces the third lower heating unit and operates at the third temperature, and the portable RT-PCR device may control the plurality of lower heating units and the plurality of upper heating units in such a manner as to maintain the temperatures at which the plurality of lower heating units and the plurality of upper heating units, respectively, operate.

### [Advantageous Effects of Invention]

In a portable RT-PCR device according to a proposed embodiment of the present disclosure, an abrupt change in temperature, such as an abrupt increase or decrease in temperature, does not take place. Thus, it is easy to perform temperature control, and a polymerase chain reaction can be smoothly performed.

In addition, the use of a membrane-type specimen unit having an aspect ratio of less than 1 decreases an entire height of the portable RT-PCR device. Thus, there is provided the advantage that the specimen unit can be input into the portable RT-PCR device in an easier manner.

### [Brief Description of Drawings]

FIG. 1 is a view illustrating a portable RT-PCR device according to a first embodiment of the present disclosure.
FIG. 2 is a view illustrating that chambers of the portable RT-PCR device in FIG. 1 are moved.
FIG. 3 is a view illustrating a state of the portable RT-PCR device in FIG. 1, when viewed from the III direction.
FIG. 4 is a view illustrating a state where an upper heat unit of the portable RT-PCR device in FIG. 3 is lifted upward.
FIG. 5 is a view illustrating a chamber unit and a specimen unit of the portable RT-PCR device in FIG. 1.
FIG. 6 is a view illustrating an RT-PCR measurement method according to a second embodiment of the present disclosure that uses the portable RT-PCR device in FIG. 1.
FIG. 7 is a view illustrating a portable RT-PCR device according to a third embodiment of the present disclosure.

### [Best Mode]

Advantages and features of the present disclosure, and methods of achieving the advantages and the features will be apparent from embodiments that will be described in detail below with reference to the accompanying drawings. However, the present disclosure is not limited to the embodiments that will be disclosed below and can be implemented in various different forms. The embodiments are only provided to make the present disclosure complete and to provide definite notice as to the scope of the present disclosure to a person of ordinary skill in the art to which the present disclosure pertains. The scope of the present disclosure should be only defined by claims.

Although used to describe various constituent elements, the terms first, second, and so on do not, of course, impose any limitation on the various constituent elements. These terms are used to distinguish one constituent component from one or more other constituent components. Therefore, a first constituent element that will be described below may of course be a second constituent element that falls within the scope of the technological idea of the present invention.

The same reference numeral throughout the specification refers to the same constituent element.

Features of various embodiments of the present disclosure may be integrated or combined severally or as a whole. It would be sufficiently understood by a person of ordinary skill that various interworking operations or driving operations are technically possible. The embodiments may be implemented independently of each other or may be implemented in conjunction with each other.

Tentative effects that are not specifically mentioned in the present specification, but can be expected from the technical features of the present disclosure are regarded as being described in the present specification. The embodiments are provided in sufficient detail to enable a person of ordinary skill in the art to practice the present disclosure. Constituent elements may be illustrated in a more exaggerated manner in the drawings than they appear when the present disclosure is practiced. A detailed description of a constituent element, when determined to unnecessarily make the gist of the present disclosure obfuscated, will be omitted or be made to be brief.

The embodiments of the present disclosure will be described in detail below with reference to the accompanying drawings.

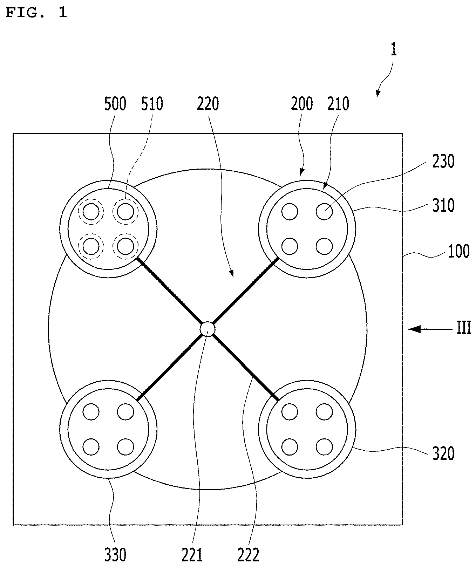

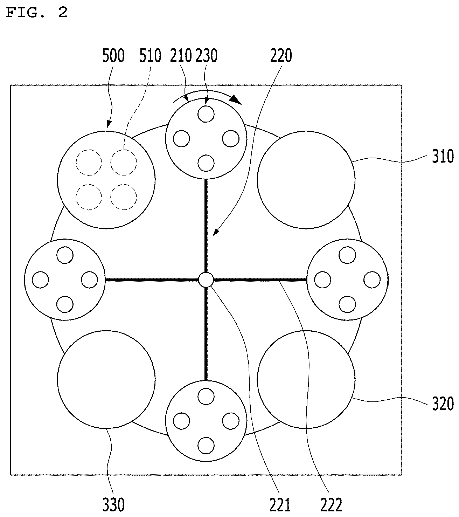

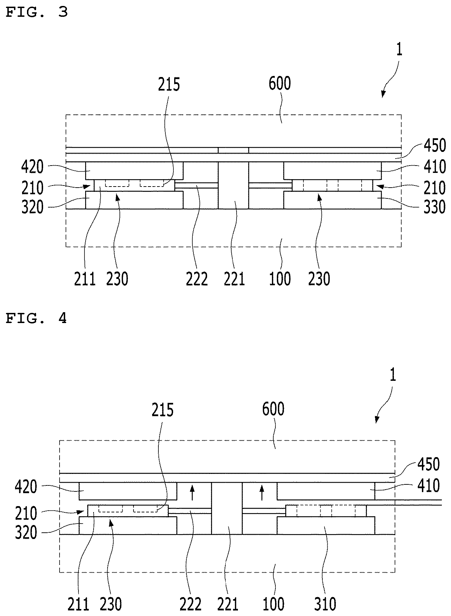

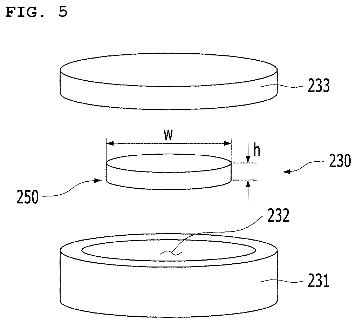

FIG. 1 is a view illustrating a portable RT-PCR device according to a first embodiment of the present disclosure. FIG. 2 is a view illustrating that chambers of the portable RT-PCR device in FIG. 1 are moved. FIG. 3 is a view illustrating a state of the portable RT-PCR device in FIG. 1, when viewed from the III direction. FIG. 4 is a view illustrating a state where an upper heat unit of the portable RT-PCR device in FIG. 3 is lifted upward. FIG. 5 is a view illustrating a chamber unit and a specimen unit of the portable RT-PCR device in FIG. 1.

With reference to FIGS. 1 to 5, in the portable RT-PCR device 1 according to the first embodiment of the present disclosure, the chambers, each accommodating a specimen unit, are moved to a plurality of heating units, respectively, each having a fixed operation temperature, and are heated. Thus, the operation temperature of the heating unit may be more uniformly maintained. As an example, the chambers may be arranged in a manner that is rotatable about a rotational center formed inside the portable RT-PCR device 1.

In addition, the specimen unit is formed in the shape of a flat membrane (for example, in the shape of a circular membrane). The specimen unit in which DNA or RNA extracted from a syringe-type portable nucleic acid extraction kit is present is simply input into the chamber unit in which a PCR solution is accommodated. With this configuration, the specimen unit may be easily input into the PCR device.

Therefore, with the portable RT-PCR device 1 according to the first embodiment of the present disclosure, it is possible to perform PCR measurement stably and smoothly in an external environment in which an urgent virus test is necessary, as well as in a laboratory environment.

The portable RT-PCR device 1 according to the first embodiment of the present disclosure includes a base unit 100, lower heating units 310, 320, and 330, a lower optical measurement unit 500, a cover unit 600, upper heating units 410 and 420, and a chamber assembly 200.

A mounting space is formed in the base unit 100, and the base unit 100 forms an exterior appearance of a lower part of the portable RT-PCR device 1. At this point, the base unit 100 may be supported on a floor surface.

The lower heating units 310, 320, and 330 are mounted in the mounting space in the base unit 100. The lower heating unit 310, 320, and 330 include a first lower heating unit 310 operating at a first temperature T₁, a second lower heating unit 320 operating at a second temperature T₂, and a third lower heating unit 330 operating at a third temperature T₃.

The first lower heating unit 310 and the third lower heating unit 320 may be arranged in such a manner that they are symmetrical about the rotational center about which the chamber assembly 200 is rotated. The second lower heating unit 320 and the lower optical measurement unit 500 may be arranged in such a manner that they are symmetrical about the rotational center. In this case, the first lower heating unit 310, the second lower heating unit 320, the third lower heating unit 330, and the lower optical measurement unit 500 are arranged around the rotational center in such a manner as to be spaced a preset space apart.

The first temperature T₁ is set to be higher than the third temperature T₃, and the third temperature T₃ is set to be higher than the second temperature T₂. Then, the first lower heating unit 310, the second lower heating unit 320, and the third lower heating unit 330 are kept at their respective set temperatures. That is, while performing the PCR measurement, the temperatures of the first lower heating unit 310, the second lower heating unit 320, and the third lower heating unit 330 remain unchanged.

In this case, as an example, the first temperature T₁ is set to approximately 97°C, and the second temperature T₂ is set to approximately 60°C. Lastly, the third temperature T₃ may be set to approximately 72°C.

The lower optical measurement unit 500 is mounted on the base unit 100 and is arranged at a different position than the lower heating units 310, 320, and 330. The lower optical measurement unit 500 provides measurement light and receives the measurement light. The lower optical measurement unit 500 may emit the measurement light to the specimen unit 250 that is positioned at a measurement-target region 510, and measure fluorescence of the specimen unit 250. At this point, a plurality of measurement-target regions 510 may be formed, and the lower optical measurement unit 500 may be a light emitting device for emitting the measurement light or an optical sensor device for receiving the measurement light. In this case, the light emitting device may be a device, such as a UV LED, that is capable of emitting light in a preset wavelength band, and the optical sensor device may a device, such as a CIS or CCD, that receives light and generates an image.

Any one specimen unit 250 that contains a testing-target specimen is moved to the first lower heating unit 310, the second lower heating unit 320, the third lower heating unit 330, and the lower optical measurement unit 500 in this order. When a step of measuring the specimen unit 250 is finished in the lower optical measurement unit 500, one measurement cycle for the specimen unit 250 is set to be ended.

In a case where the specimen unit 250 is heated by the first lower heating unit 310 that operates at the first temperature T₁, a DNA denaturation step may be performed. In a case where the specimen unit 250 is heated by the second lower heating unit 320 that operates at the second temperature T₂, a DNA annealing step may be performed. In a case where the specimen unit 250 is heated by the third lower heating unit 330 that operates at the third temperature T₃, a DNA elongation step may be performed.

The specimen unit 250 is heated by the third lower heating unit 330 for a maintenance time Tₘ. Then the specimen unit 250 is moved toward the lower optical measurement unit 500, and the PCR measurement is performed on the specimen unit 250.

The chamber assembly 200 includes a plurality of chambers 210 and a chamber movement unit 220. The plurality of chambers 210 are seated on the lower heating units 310, 320, and 330 and the lower optical measurement unit 500, respectively. The chamber movement unit 220 serves to move the plurality of chambers 210.

The chamber 210 is provided in a manner that is movable from one of the lower heating units 310, 320, and 330 to other one of the lower heating units 310, 320, and 330 or to the lower optical measurement unit 500. The plurality of chambers 210 are arranged to be spaced a preset distance apart around the rotational center formed in the base unit 100. The chamber movement unit 220 rotates the plurality of chambers 210 about the rotational center in one direction at the same time. In the present embodiment, as an example, the chamber movement unit 220 may rotate the chambers 210 clockwise, and the first lower heating unit 310, the second lower heating unit 320, the third lower heating unit 330, and the lower optical measurement unit 500 may be arranged in this order in the clockwise direction.

The chamber 210 includes a chamber body 211 for accommodating a chamber unit 230 inside which a specimen-unit accommodation space in which the specimen unit 250 is accommodated is formed. In this case, the chamber body 211 may be formed in the shape of a plate having a width greater than a height in the vertical direction, and a chamber-unit insertion space 215 into which the chamber unit 230 is to be inserted is formed in the chamber body 211. The chamber-unit insertion space 215 is formed in such a manner as to have a width corresponding to a width of each of the chamber units 230. In a state where the chamber unit 230 is inserted into the chamber-unit insertion space 215 in the chamber 210, lower and side surfaces of the chamber unit 230 are completely brought into close contact with an inner wall of the chamber-unit insertion space 215. In order to facilitate transfer of heat toward the chamber unit 230, the chamber 210 may be formed of a material, such as a metal, that has a high heat transfer coefficient.

The chamber unit 230 includes a chamber unit body 211 in which the specimen-unit accommodation space 232 is formed, and a cap unit 233 that covers the specimen-unit accommodation space 232 in the chamber unit body 211 from above.

In this time, the specimen unit 250 is formed in such a manner as to have an aspect ratio, that is, a height-to-width ratio, which is greater than 0 and smaller than 1. As an example, the specimen unit 250 may be formed with a disk-shaped membrane structure formed of a porous material.

A measurement solution that is the PCR solution is accommodated in the specimen-unit accommodation space 231 in each of the chamber units 230. The specimen-unit accommodation space 231 is formed in such a manner as to have an aspect ratio that is greater than 0 and smaller than 1. That is, the specimen-unit accommodation space 231 is formed in such a manner as to have a width greater than a height in the upward-downward direction. The specimen unit accommodation space 231 is formed in such a manner as to have a volume of 20 µl to 100 µl. As an example, in the present embodiment, a measurement solution of approximately 50 µl is accommodated in the specimen-unit accommodation space 231, and the specimen unit 250 is immersed in the measurement solution in such a manner as to be impregnated therewith.

The chamber movement unit 220 includes a plurality of connection brackets 222 and a rotation shaft 221. First end portions of the plurality of connection brackets 222 are connected to the chambers 210, respectively. The rotation shaft 221 is provided in a manner that is rotatable about the rotational center. Second end portions of the plurality of connection brackets 222 are connected to the rotation shaft 221.

The rotation of the chamber movement unit 220 is stopped for the maintenance time Tₘ for which the chambers 210 are seated on the lower heating units 310, 320, and 330, respectively. The chamber movement unit 220 is rotated for a movement time Tᵣ from when the maintenance time Tₘ expires to when a next maintenance time Tₘ starts. In this case, the maintenance time Tₘ is set to be longer than the movement time Tᵣ.

FIGS. 3 and 4 illustrate an internal configuration of the portable RT-PCR device 1, when viewed from the side in a state where the cover unit 600 of the portable RT-PCR device 1 according to the first embodiment of the present disclosure covers the mounting space in the base unit 100.

More specifically, the cover unit 600 is arranged over the base unit 100, covers the mounting space in the base unit 100 from above, and forms an exterior appearance of an upper portion of the portable RT-PCR device 1.

The upper heating units 410 and 420 and the upper heating unit (not illustrated) are arranged between the lower heating unit 310 and the cover unit 600, between the lower heating unit 320 and the cover unit 600, and between the lower heating unit 330 and the cover unit 600, respectively. In this case, the upper heating units 410 and 420 and the upper heating unit (not illustrated) are selectively brought into contact with upper surfaces, respectively, of the chambers 210. The upper heating units 410 and 420 and the upper heating unit (not illustrated) are formed in such a manner that shapes thereof correspond to shapes, respectively, of the lower heating units 310, 320, and 330. The upper heating units 410 and 420 and the upper heating unit (not illustrated ) include a first upper heating unit 410, a second upper heating unit 420, and a third upper heating unit (not illustrated) that correspond to the first lower heating unit 310, the second lower heating unit 320, and the third lower heating unit 330, respectively. The upper heating units 410 and 420 and the upper heating unit (not illustrated) operate after heated to temperatures, respectively, that are the same as those of their respective corresponding lower heating units 310, 320, and 330. The chamber 210 is arranged between each of the lower heating units 310, 320, and 330 and each of the upper heating units 410 and 420 and the upper heating unit (not illustrated) in a manner that is brought into close contact with the lower heating unit and the upper heating unit. Thus, the chamber unit 230 may be heated in a more stable state.

The upper heating units 410 and 420 and the upper heating unit (not illustrated) and the lower heating units 310, 320, and 330 of the portable RT-PCR device 1 according to the first embodiment are formed in such a manner that a distance between each of the upper heating units 410 and 420 and the upper heating unit (not illustrated) and each of the lower heating units 310, 320, and 330 is variable. That is, when a heated state of the chamber 210 is attained on a per-step basis, the chamber 210 is moved toward other upper heating units 410 and 420 and upper heating unit (not illustrated) and other lower heating units 310, 320, and 330. Accordingly, the distance between each of the upper heating units 410 and 420 and the upper heating unit (not illustrated) and each of the lower heating units 310, 320, and 330 varies in such a manner as to facilitate movements to other upper heating units 410 and 420 and upper heating unit (not illustrated) and other lower heating units 310, 320, and 330.

More specifically, in a case where each of the chambers 210 is arranged between each of the upper heating units 410 and 420 and the upper heating unit (not illustrated) and each of the lower heating units 310, 320, and 330 and is not moved for the maintenance time, the distance between each of the upper heating units 410 and 420 and the upper heating unit (not illustrated) and each of the lower heating units 310, 320, and 330 corresponds to a height of each of the chambers 210. That is, the chamber 210 is brought into close contact with each of the upper heating units 410 and 420 and the upper heating unit (not illustrated) and each of the lower heating units 310, 320, and 330 and thus heat may be stably supplied to the chamber unit 230.

In a case where the maintenance time expires and where the chambers 210 are moved toward other upper heating units 410 and 420 and upper heating unit (not illustrated), respectively, and toward other lower heating units 310, 320, and 330, respectively, the distance between each of the upper heating units 410 and 420 and the upper heating unit (not illustrated) and each of the lower heating unit 310, 320, and 330 is set to be greater than the height of each of the chambers 210. That is, the chamber 210 is no longer in contact with each of the upper heating units 410 and 420 and the upper heating unit (not illustrated) and each of the lower heating units 310, 320, and 330. Thus, the movement of the chamber 210 is facilitated.

In the present embodiment, the upper heating units 410 and 420 and the upper heating unit (not illustrated) may be formed in such a manner that they are connected to an upper-heating-unit movement unit 450 that is arranged in a manner that is movable in the upward-downward direction and are movable at the same time in the upward-downward direction. In the first embodiment of the present disclosure, a configuration may also be employed where the lower heating units 310, 320, and 330 are moved in the upward-downward direction and thus where the distance between each of the upper heating units 410 and 420 and the upper heating unit (not illustrated) and each of the lower heating units 310, 320, and 330 varies.

An upper optical measurement unit faces the lower optical measurement unit 500. The upper optical measurement unit receives the measurement light emitted from the lower optical measurement unit 500 or provides the measurement light toward the lower optical measurement unit 500. That is, the upper optical measurement unit and the lower optical measurement unit 500 are provided in a pair in such a manner that they correspond to each other. By emitting or receiving light, the upper optical measurement unit and the lower optical measurement unit 500 perform fluorescence measurement on the specimen units 250 that are accommodated in the chamber units 230, respectively, that are arranged in the upper optical measurement unit and the lower optical measurement unit 500.

In the present embodiment, a configuration may be employed where the upper heating units 410 and 420 and the upper heating unit (not illustrated) and the upper optical measurement unit are mounted in the cover unit 600.

An RT-PCR measurement method according to a second embodiment of the present disclosure that uses the portable RT-PCR device will be described in more detail below.

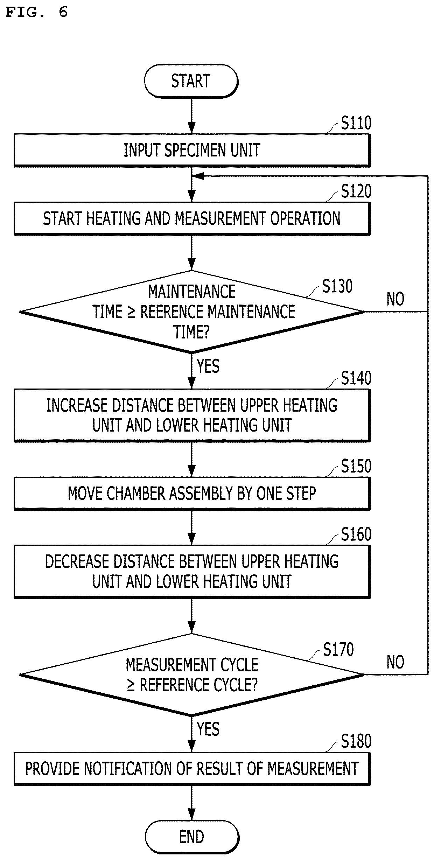

FIG. 6 is a view illustrating the RT-PCR measurement method that uses the portable RT-PCR device in FIG. 1.

With reference to FIG. 6, in the RT-PCR measurement method according to the second embodiment of the present disclosure, first, a specimen unit input step S110 of accommodating the chamber units 230, in each of which the specimen unit 250 is accommodated, into the chambers 210, respectively, is performed.

Next, in a state where the specimen unit 250 is input, a heating and measurement operation starting step S120 of performing a heating or measurement operation on the specimen unit 250 is performed.

At this point, the lower heating units 310, 320, and 330 and the upper heating units 410 and 420 and the upper heating unit (not illustrated), respectively, are controlled in such a manner as to maintain temperatures at which they, respectively, operate. At this point, the first lower heating unit 310 and the first upper heating unit 410 operate at the first temperature T₁, the second lower heating unit 320 and the second upper heating unit 420 operate at the second temperature T₂, and the third lower heating unit 330 and the third upper heating unit operate at the third temperature T₃. Then, a fluorescence measurement operation is performed on the chamber 210 that is arranged between the upper optical measurement unit and the lower optical measurement unit 500.

Next, in a case where the maintenance time Tₘ in the heating and measurement operation starting step S120 is longer than or the same as a preset reference maintenance time T_{m,r} (S130), a distance-between-heating-units increasing step S140 of increasing the distance between each of the upper heating units 410 and 420 and the upper heating unit (not illustrated) and each of the lower heating units 310, 320, and 330 is performed.

In the distance-between-heating-units increasing step S140, the upper heating units 410 and 420 and the lower heating units 310 and 320 are formed in such a manner that the distance between each of the upper heating units 410 and 420 and each of the lower heating units 310 and 320 is greater than the height of each of the chambers 210. In the present embodiment, the distance between each of the upper heating units 410 and 420 and each of the lower heating units 310 and 320 may be increased by lifting the upper heating units 410 and 420.

Next, a chamber assembly one-step-movement step S150 of moving the plurality of chambers 210 by one step is performed. In the chamber-assembly one-step-movement step S150, the chambers 210 are rotated by a preset angle about the rotational center formed in the base unit 100. In the present embodiment, the chambers 210 are rotated clockwise by approximately 90°.

After the chamber-assembly one-step movement step S150 is performed, a distance-between-heating-units deceasing step S160 of decreasing the distance between each of the upper heating units 410 and 420 and the upper heating unit (not illustrated) and each of the lower heating units 310, 320, and 330 is performed. In the distance-between-heating-units deceasing step S160, the distance between each of the upper heating units 410 and 420 and each of the lower heating units 310 and 320 corresponds to the height of each of the chambers 210. In the present embodiment, the upper heating units 410 and 420 may descend.

Next, in a case where a measurement cycle for the plurality of chambers 210 is set to a preset reference cycle or above (S170), a measurement result notification step S180 of providing notification of a result of measurement is performed. At this point, one measurement cycle is defined as four steps by which the chamber 210 is moved.

In a case where the maintenance time in the heating and measurement operation starting step S120 is shorter than a preset reference maintenance time (S130), the heating and measurement operation starting step S120 is performed.

In addition, in a case where the measurement cycle for the plurality of chambers 210 is shorter than a preset reference cycle (S170), the heating and measurement operation starting step S120 is re-performed.

According to the proposed embodiment, the temperature is easy to control, and the polymerase chain reaction may be smoothly performed. In addition, the use of a membrane-type specimen unit having an aspect ratio of less than 1 decreases an entire height of the portable RT-PCR device. Thus, there is provided the advantage that the specimen unit can be input into the portable RT-PCR device in an easier manner.

The configuration where the chamber 210 is directly connected to the rotation shaft 221 and is connected to the linearly formed connection bracket 222 is described as being employed in the first embodiment. However, in the embodiment of the present disclosure, a configuration may also be employed where the chamber 210 is rotatably arranged using a chamber connection bracket connecting the chambers 210 to each other and a connection member connecting the chamber connection bracket to the rotation shaft 221.

In addition, in the first embodiment of the present disclosure, a configuration may also be employed where a first end portion of the rotation shaft 221 is rotatably connected to the base unit 100, where a second end portion thereof is connected to the cover unit 600 detachably and rotatably, and thus where the rotation shaft 221 is more stably rotated.

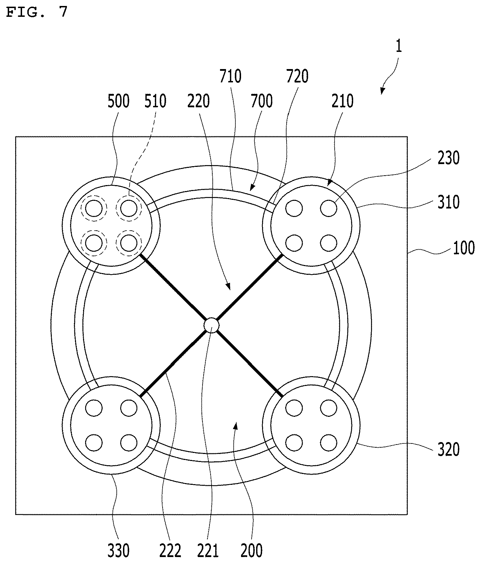

FIG. 7 is a view illustrating a portable RT-PCR device 1 according to a third embodiment of the present disclosure.

A configuration of the portable RT-PCR device 1 according to the third embodiment is substantially the same as the configuration of the portable RT-PCR device 1 illustrated in FIGS. 1 to 6, except that a guide unit for guiding the movement of the chamber 210 is arranged. Therefore, a constituent element of the portable RT-PCR device 1 according to the third embodiment that has a characteristic feature will be mostly described below.

With reference to FIG. 7, the portable RT-PCR device 1 according to the third embodiment of the present disclosure further includes a guide unit 700 for guiding rotational movement of the chamber 210.

More specifically, the guide unit 700 includes a first guide unit 710 and a second guide unit 720. The first guide unit 710 is arranged to be positioned between one of the lower heating units 310, 320, and 330 and other one of the lower heating units 310, 320, and 330 or the lower optical measurement unit 500 and serves to guide the rotation of the chambers 210. The second guide unit 720 is formed to be positioned on upper surfaces of the lower heating units 310, 320, and 330 and the lower optical measurement unit 500.

The first guide unit 710 is formed in such a manner as to have a curvature corresponding to an a curvature radius of an imaginary circle that is formed when the chambers 210 are rotated. A guide groove or a guide protrusion that is engaged with the first guide unit 710 is formed in or on a lower portion of each of the chamber 210. The first guide unit 710 may be formed in the shape of a protrusion or groove in such a manner as to correspond to the guide groove in each of the chambers 210 or the guide protrusion thereon.

The second guide unit 720 is connected to the first guide unit 710 and has the same curvature radius as the first guide unit 710. The second guide unit 720 is formed in a manner that is selectively engaged with the guide groove in each of the chambers 210 or the guide protrusion thereon.

In the proposed embodiment, the guide unit 700 guides the rotation of the chambers 210. Thus, there is provided the advantage that the chambers 210 are stably rotated.

The desired embodiments of the present disclosure are described above, but the present disclosure is not limited thereto. Various modifications are possibly made to the embodiments of the present disclosure without departing from the claims.

### [Mode for Invention]

A mode for practicing the present disclosure is described above under Best Mode.

### [Industrial Applicability]

The present disclosure relates to a portable RT-PCR device and an RT-PCR measurement method that uses the portable RT-PCR device. The portable RT-PCR device has operability and industrial applicability in the medical field.

## Claims

1. A portable RT-PCR device (1) comprising:
a base unit (100) in which a mounting space is formed;
a plurality of lower heating units (310, 320, 330) mounted in the mounting space in the base unit (100);
a lower optical measurement unit (500) mounted in the base unit (100) and arranged in a different position than the plurality of lower heating units (310, 320, 330) and providing measurement light or receiving the measurement light; and
a chamber assembly (200) including a plurality of chambers (210) that are seated on the plurality of lower heating units (310, 320, 330) and the lower optical measurement unit (500), respectively, each chamber (210) being provided in such a manner to be movable from one of the plurality of lower heating units (310, 320, 330) to other one of the plurality of lower heating units (310, 320, 330) or to the lower optical measurement unit (500),
wherein each of the plurality of chambers (210) comprises:
a chamber body (211) for accommodating a chamber unit (230) in which a specimen-unit accommodation space (232) inside which a specimen unit (250) is accommodated is formed,
**characterized in that** the chamber body (211) is formed to receive the chamber unit (230) having a chamber unit body (231) in which the specimen-unit accommodation space (232) is formed to accommodate the specimen unit (250) having an aspect ratio, that is, a height-to-width ratio, which is greater than 0 and smaller than 1; and a cap unit (233) covering the specimen-unit accommodation space (232) in the chamber unit body (231) from above, and
wherein the portable RT-PCR device (1) further comprises
a cover unit (600) arranged over the base unit (100) and covering the mounting space;
a plurality of upper heating units (410, 420) each of which is arranged between each of the plurality of lower heating units (310, 320, 330) and the cover unit (600) and which are selectively brought into contact with upper surfaces, respectively, of the plurality of chambers (210); and
an upper optical measurement unit facing the lower optical measurement unit (500), receiving the measurement light emitted from the lower optical measurement unit (500) or providing the measurement light toward the lower optical measurement unit (500); and
wherein the plurality of upper heating units (410, 420) and the plurality of lower heating units (310, 320, 330) are formed in such a manner that a distance between each of the plurality of upper heating units (410, 420) and each of the plurality of lower heating units (310, 320, 330) is variable,
wherein, in a case where each of the plurality of chambers (210) is arranged between each of the plurality of upper heating units (410, 420) and each of the plurality of lower heating units (310, 320, 330) and is not moved for a maintenance time (Tₘ), the distance between each of the plurality of upper heating units (410, 420) and each of the plurality of lower heating units (310, 320, 330) corresponds to a height of each of the plurality of chambers (210), and
wherein, in a case where the maintenance time (Tₘ) expires and where the plurality of chambers (210) are moved toward other upper heating units (410, 420), respectively, and toward other lower heating units (310, 320, 330), respectively, the distance between each of the plurality of upper heating units (410, 420) and each of the plurality of lower heating units (310, 320, 330) is set to be greater than the height of each of the plurality of chambers (210).

2. The portable RT-PCR device (1) of claim 1, wherein the chamber assembly (200) further comprises:
a chamber movement unit (220) for moving the plurality of chambers (210),
wherein the plurality of chambers (210) are arranged around a rotational center formed in the base unit (100) in such a manner as to be spaced a preset distance apart, and
wherein the chamber movement unit (220) rotates the plurality of chambers (210) around the rotational center in one direction at the same time.

3. The portable RT-PCR device (1) of claim 2, wherein the chamber movement unit (220) comprises:
a plurality of connection brackets (222), first end portions of which are connected to the plurality of chambers (210), respectively; and
a rotation shaft (221) to which second end portions of the plurality of connection brackets (222) are connected and which is provided in a manner that is rotatable about the rotational center,
wherein the rotation of the chamber movement unit (220) is configured to be stopped for a maintenance time (Tₘ), and the chamber movement unit (220) is configured to rotate for a movement time(Tᵣ), and
wherein the maintenance time (Tₘ) is set to be longer than the movement time (Tᵣ).

4. The portable RT-PCR device (1) of claim 3, wherein a first guide unit (710) for guiding the rotation of each of the plurality of chambers (210) is formed to be positioned between one of the plurality of lower heating units (310, 320, 330) and other one of the plurality of lower heating units (310, 320, 330) or the lower optical measurement unit (500),
wherein the first guide unit (710) is formed in such a manner as to have a curvature corresponding to a curvature radius of an imaginary circle that is formed when the plurality of chambers (210) are rotated, and
wherein a guide groove or a guide protrusion that is engaged with the first guide unit (710) is formed in or on a lower portion of each of the plurality of chambers (210).

5. The portable RT-PCR device (1) of claim 4, wherein a second guide unit (720) that is connected to the first guide unit (710), has the same curvature radius as the first guide unit (710), and is selectively engaged with the guide groove in each of the plurality of chambers (210) or the guide protrusion thereon is formed to be positioned on upper surfaces of the lower heating unit (310, 320, 330) and the lower optical measurement unit (500).

6. The portable RT-PCR device (1) of claim 2, wherein the lower heating unit (310, 320, 330) comprises:
a first lower heating unit (310) configured to operate at a first temperature(T₁);
a second lower heating unit (320) configured to operate at a second temperature (T₂); and
a third lower heating unit (330) configured to operate at a third temperature (T₃), and
wherein the first lower heating unit (310) and the third lower heating unit (330) are symmetrical about the rotational center, and the second lower heating unit (320) and the lower optical measurement unit (500) are symmetrical about the rotational center.

7. The portable RT-PCR device (1) of claim 6, wherein the first temperature (T₁) is set to be higher than the third temperature (T₃), and the third temperature (T₃) is set to be higher than the second temperature (T₂), and
wherein the first lower heating unit (310), the second lower heating unit (320), and the third lower heating unit (330) are configured to be kept at their respective set temperatures.

8. The portable RT-PCR device (1) of claim 1, wherein the plurality of lower heating units (310, 320, 330) each are formed in the shape of a plate,
wherein lower surfaces of the plurality of chambers (210) are brought into full contact with the plurality of lower heating units (310, 320, 330), respectively,
wherein a chamber-unit insertion space (215) into which the chamber unit (230) is to be inserted is formed in the chamber body (211) of each of the plurality of chambers (210), and
wherein the chamber-unit insertion space (215) is formed in such a manner that a width thereof corresponds to a width of each of the plurality of chamber units (230).

9. The portable RT-PCR device (1) of claim 1, wherein a measurement solution is accommodated in the specimen-unit accommodation space (232) in each of the plurality of chamber units (230), and the specimen-unit accommodation space (232) is formed in such a manner that the aspect ratio thereof is greater than 0 and smaller than 1, and
wherein the specimen-unit accommodation space (232) is formed in such a manner that a volume thereof is 20 µl to 100 µl.

10. The portable RT-PCR device (1) of claim 1, wherein the specimen unit (250) is formed with a membrane structure formed of a porous material.

11. An RT-PCR measurement method that uses the portable RT-PCR device (1) of claim 1, the method comprising:
a specimen-unit input step (S110) of accommodating a plurality of chamber units (230), in each of which the specimen unit (250) is accommodated, into the plurality of chambers (210), respectively;
a heating and measurement operation starting step (S120) of performing a heating or measurement operation on the specimen unit (250) in a state where the specimen unit (250) is input;
a chamber-assembly one-step movement step (S150) of moving the plurality of chambers (210) by one step in a case where a maintenance time (Tₘ) in the heating and measurement operation starting step (S120) is longer than a preset reference maintenance time (S130); and
a measurement result notification step (S180) of providing notification of a result of measurement in a case where a measurement cycle for the plurality of chambers (210) is set to be longer than a preset reference cycle (S170),
wherein one measurement cycle is defined as four steps by which each of the plurality of chambers (210) is moved.

12. The RT-PCR measurement method of claim 11, further comprising:
a distance-between-heating-units increasing step (S140) of increasing a distance between each of the plurality of upper heating units (410, 420) that are arranged to face the plurality of lower heating units (310, 320, 330), respectively, and each of the plurality of lower heating units (310, 320, 330), before the chamber-assembly one-step movement step (S150) is performed, in a case where the maintenance time in the heating and measurement operation starting step (S120) is longer than a preset reference maintenance time (S130); and
a distance-between-heating-units decreasing step (S160) of decreasing the distance between each of the plurality of upper heating units (410, 420) and each of the plurality of lower heating units (310, 320, 330) after the chamber-assembly one-step movement step (S150) is performed,
wherein in the distance-between-heating-units increasing step (S140), the plurality of upper heating units (410, 420) and the plurality of lower heating units (310, 320, 330) are formed in such a manner that the distance between each of the plurality of upper heating units (410, 420) and each of the plurality of lower heating units (310, 320, 330) is greater than a height of each of the plurality of chambers (210), and
wherein in the distance-between-heating-units deceasing step (S160), the distance between each of the plurality of upper heating units (410, 420) and each of the plurality of lower heating units (310, 320, 330) corresponds to the height of each of the plurality of chambers (210).

13. The RT-PCR measurement method of claim 11, wherein in the chamber-assembly one-step movement step (S150), the plurality of chambers (210) are rotated by a preset angle about a rotational center formed in the base unit (100),
wherein the plurality of lower heating units (310, 320, 330) include a first lower heating unit (310) that operates at a first temperature (T₁), a second lower heating unit (320) that operates at a second temperature (T₂), and a third lower heating unit (330) that operates at a third temperature (T₃),
wherein the plurality of upper heating units (410, 420) that face the plurality of lower heating units (310, 320, 330), respectively, include a first upper heating unit (410) that faces the first lower heating unit (310) and operates at the first temperature (T₁), a second upper heating unit (420) that faces the second lower heating unit (320) and operates at the second temperature (T₂), and a third upper heating unit that faces the third lower heating unit (330) and operates at the third temperature (T₃), and
wherein the portable RT-PCR device (1) controls the plurality of lower heating units (310, 320, 330) and the plurality of upper heating units (410, 420) in such a manner as to maintain the temperatures at which the plurality of lower heating units (310, 320, 330) and the plurality of upper heating units (410, 420), respectively, operate.

## Patentansprüche

1. Tragbare RT-PCR-Vorrichtung (1), Folgendes umfassend:
eine Basiseinheit (100), in der ein Bestückungsraum ausgebildet ist;
eine Vielzahl unterer Heizeinheiten (310, 320, 330), die in dem Bestückungsraum in der Basiseinheit (100) befestigt sind;
eine untere optische Messeinheit (500), die in der Basiseinheit (100) befestigt und in einer anderen Position als die Vielzahl unterer Heizeinheiten (310, 320, 330) angeordnet ist und Messlicht bereitstellt oder das Messlicht empfängt; und
eine Kammeranordnung (200), die eine Vielzahl Kammern (210) umfasst, die jeweils auf der Vielzahl unterer Heizeinheiten (310, 320, 330) und der unteren optischen Messeinheit (500) sitzen, wobei jede Kammer (210) derartig bereitgestellt ist, dass sie von einer der Vielzahl unterer Heizeinheiten (310, 320, 330) zu einer anderen der Vielzahl unterer Heizeinheiten (310, 320, 330) oder zu der unteren optischen Messeinheit (500) beweglich ist,
wobei jede der Vielzahl Kammern (210) Folgendes umfasst:
einen Kammerkörper (211) zum Unterbringen einer Kammereinheit (230), in der ein Probeneinheit-Unterbringungsraum (232) ausgebildet ist, in dem eine Probeneinheit (250) untergebracht ist,
**dadurch gekennzeichnet, dass** der Kammerkörper (211) ausgebildet ist, um die Kammereinheit (230) aufzunehmen, die einen Kammereinheitskörper (231) aufweist, in dem der Probeneinheit-Unterbringungsraum (232) ausgebildet ist, um die Probeneinheit (250) aufzunehmen, die ein Seitenverhältnis, d. h. ein Höhe-zu-Breite-Verhältnis aufweist, das größer als 0 und kleiner als 1 ist; und
eine Kappeneinheit (233) aufweist, die den Probeneinheit-Unterbringungsraum (232) in dem Kammereinheitskörper (231) von oben bedeckt, und
wobei die tragbare RT-PCR-Vorrichtung (1) weiterhin Folgendes umfasst
eine Deckeinheit (600), die über der Basiseinheit (100) angeordnet ist und den Bestückungsraum bedeckt;
eine Vielzahl oberer Heizeinheiten (410, 420), die jeweils zwischen jeder der Vielzahl unterer Heizeinheiten (310, 320, 330) und der Deckeinheit (600) angeordnet sind und die selektiv jeweils mit oberen Oberflächen der Vielzahl Kammern (210) in Kontakt gebracht werden; und
eine obere optische Messeinheit, die der unteren optischen Messeinheit (500) zugewandt ist, die das von der unteren optischen Messeinheit (500) emittierte Messlicht empfängt oder das Messlicht in Richtung der unteren optischen Messeinheit (500) bereitstellt; und
wobei die Vielzahl oberer Heizeinheiten (410, 420) und die Vielzahl unterer Heizeinheiten (310, 320, 330) derartig ausgebildet sind, dass ein Abstand zwischen jeder der Vielzahl oberer Heizeinheiten (410, 420) und jeder der Vielzahl unterer Heizeinheiten (310, 320, 330) variabel ist,
wobei in einem Fall, in dem jede der Vielzahl Kammern (210) zwischen jeder der Vielzahl oberer Heizeinheiten (410, 420) und jeder der Vielzahl unterer Heizeinheiten (310, 320, 330) angeordnet ist und für eine Beibehaltungszeit (Tₘ) nicht bewegt wird, der Abstand zwischen jeder der Vielzahl oberer Heizeinheiten (410, 420) und jeder der Vielzahl unterer Heizeinheiten (310, 320, 330) einer Höhe jeder der Vielzahl Kammern (210) entspricht und
wobei in einem Fall, in dem die Beibehaltungszeit (Tₘ) abläuft und in dem die Vielzahl Kammern (210) jeweils zu anderen oberen Heizeinheiten (410, 420) und jeweils zu anderen unteren Heizeinheiten (310, 320, 330) hin bewegt wird, der Abstand zwischen jeder der Vielzahl oberer Heizeinheiten (410, 420) und jeder der Vielzahl unterer Heizeinheiten (310, 320, 330) derartig eingestellt ist, dass er größer ist als die Höhe jeder der Vielzahl Kammern (210).

2. Tragbare RT-PCR-Vorrichtung (1) nach Anspruch 1, wobei die Kammeranordnung (200) weiterhin Folgendes umfasst:
eine Kammerbewegungseinheit (220) zum Bewegen der Vielzahl Kammern (210),
wobei die Vielzahl Kammern (210) um ein Rotationszentrum herum angeordnet sind, das in der Basiseinheit (100) derartig ausgebildet ist, dass sie einen voreingestellten Abstand voneinander beabstandet sind, und
wobei die Kammerbewegungseinheit (220) die Vielzahl Kammern (210) gleichzeitig in eine Richtung um das Rotationszentrum herum dreht.

3. Tragbare RT-PCR-Vorrichtung (1) nach Anspruch 2, wobei die Kammerbewegungseinheit (220) Folgendes umfasst:
eine Vielzahl Verbindungshalterungen (222), deren erste Endabschnitte jeweils mit der Vielzahl Kammern (210) verbunden sind; und
eine Rotationswelle (221), mit der zweite Endabschnitte der Vielzahl Verbindungshalterungen (222) verbunden sind und die derartig bereitgestellt ist, dass sie um das Rotationszentrum herum drehbar ist,
wobei die Rotation der Kammerbewegungseinheit (220) eingerichtet ist, um für eine Beibehaltungszeit (Tₘ) gestoppt zu werden, und die Kammerbewegungseinheit (220) eingerichtet ist, um für eine Bewegungszeit (Tᵣ) zu rotieren, und
wobei die Beibehaltungszeit (Tₘ) länger eingestellt ist als die Bewegungszeit (Tᵣ).

4. Tragbare RT-PCR-Vorrichtung (1) nach Anspruch 3, wobei eine erste Führungseinheit (710) zum Führen der Rotation jeder der Vielzahl Kammern (210) so ausgebildet ist, dass sie zwischen einer der Vielzahl unterer Heizeinheiten (310, 320, 330) und einer anderen der Vielzahl unterer Heizeinheiten (310, 320, 330) oder der unteren optischen Messeinheit (500) angeordnet ist,
wobei die erste Führungseinheit (710) derartig ausgebildet ist, dass sie eine Krümmung aufweist, die einem Krümmungsradius eines imaginären Kreises entspricht, der ausgebildet wird, wenn die Vielzahl Kammern (210) gedreht werden, und
wobei eine Führungsnut oder ein Führungsvorsprung, der mit der ersten Führungseinheit (710) in Eingriff steht, in oder auf einem unteren Abschnitt jeder der Vielzahl Kammern (210) ausgebildet ist.

5. Tragbare RT-PCR-Vorrichtung (1) nach Anspruch 4, wobei eine zweite Führungseinheit (720), die mit der ersten Führungseinheit (710) verbunden ist, den gleichen Krümmungsradius wie die erste Führungseinheit (710) aufweist und selektiv mit der Führungsnut in jeder der Vielzahl Kammern (210) oder dem Führungsvorsprung darauf in Eingriff steht, so ausgebildet ist, dass sie auf oberen Oberflächen der unteren Heizeinheit (310, 320, 330) und der unteren optischen Messeinheit (500) angeordnet ist.

6. Tragbare RT-PCR-Vorrichtung (1) nach Anspruch 2, wobei die untere Heizeinheit (310, 320, 330) Folgendes umfasst:
eine erste untere Heizeinheit (310), die eingerichtet ist, um bei einer ersten Temperatur (T₁) zu arbeiten;
eine zweite untere Heizeinheit (320), die eingerichtet ist, um bei einer zweiten Temperatur (T₂) zu arbeiten; und
eine dritte untere Heizeinheit (330), die eingerichtet ist, um bei einer dritten Temperatur (T₃) zu arbeiten, und
wobei die erste untere Heizeinheit (310) und die dritte untere Heizeinheit (330) symmetrisch um das Rotationszentrum sind und die zweite untere Heizeinheit (320) und die untere optische Messeinheit (500) symmetrisch um das Rotationszentrum sind.

7. Tragbare RT-PCR-Vorrichtung (1) nach Anspruch 6, wobei die erste Temperatur (T₁) höher eingestellt ist als die dritte Temperatur (T₃), und die dritte Temperatur (T₃) höher eingestellt ist als die zweite Temperatur (T₂), und wobei die erste untere Heizeinheit (310), die zweite untere Heizeinheit (320) und die dritte untere Heizeinheit (330) eingerichtet sind, um auf ihren jeweiligen eingestellten Temperaturen gehalten zu werden.

8. Tragbare RT-PCR-Vorrichtung (1) nach Anspruch 1, wobei die Vielzahl unterer Heizeinheiten (310, 320, 330) jeweils in der Gestalt einer Platte ausgebildet sind,
wobei untere Oberflächen der Vielzahl Kammern (210) jeweils in vollen Kontakt mit der Vielzahl unterer Heizeinheiten (310, 320, 330) gebracht werden,
wobei ein Kammereinheit-Einsetzraum (215), in den die Kammereinheit (230) eingesetzt werden soll, in dem Kammerkörper (211) jeder der Vielzahl Kammern (210) ausgebildet ist, und
wobei der Kammereinheit-Einsetzraum (215) derartig ausgebildet ist, dass seine Breite einer Breite jeder der Vielzahl Kammereinheiten (230) entspricht.

9. Tragbare RT-PCR-Vorrichtung (1) nach Anspruch 1, wobei eine Messlösung in dem Probeneinheit-Unterbringungsraum (232) in jeder der Vielzahl Kammereinheiten (230) untergebracht ist und der Probeneinheit-Unterbringungsraum (232) derartig ausgebildet ist, dass sein Seitenverhältnis größer als 0 und kleiner als 1 ist und
wobei der Probeneinheit-Unterbringungsraum (232) derartig ausgebildet ist, dass sein Volumen 20 µl bis 100 µl ist.

10. Tragbare RT-PCR-Vorrichtung (1) nach Anspruch 1, wobei die Probeneinheit (250) mit einer Membranstruktur ausgebildet ist, die aus einem porösen Material ausgebildet ist.

11. RT-PCR-Messverfahren, das die tragbare RT-PCR-Vorrichtung (1) nach Anspruch 1 verwendet, das Verfahren Folgendes umfassend:
einen Probeneinheit-Einsetzschritt (S110) des Unterbringens einer Vielzahl Kammereinheiten (230), in denen jeweils die Probeneinheit (250) untergebracht ist, jeweils in der Vielzahl Kammern (210);
einen Heiz- und Messbetriebsstartschritt (S120) des Durchführens eines Heiz- oder Messbetriebs an der Probeneinheit (250) in einem Zustand, in dem die Probeneinheit (250) eingesetzt wird;
einen einstufigen Bewegungsschritt der Kammeranordnung (S150) des Bewegens der Vielzahl Kammern (210) um einen Schritt in einem Fall, in dem eine Beibehaltungszeit (Tₘ) in dem Heiz- und Messbetriebsstartschritt (S120) länger ist als eine voreingestellte Referenzbeibehaltungszeit (S130); und
einen Messergebnisbenachrichtigungsschritt (S180) des Bereitstellens einer Benachrichtigung über ein Messergebnis in einem Fall, in dem ein Messzyklus für die Vielzahl Kammern (210) so eingestellt ist, dass er länger ist als ein voreingestellter Referenzzyklus (S170),
wobei ein Messzyklus als vier Schritte definiert ist, durch die jede der Vielzahl Kammern (210) bewegt wird.

12. RT-PCR-Messverfahren nach Anspruch 11, weiterhin Folgendes umfassend:
einen Schritt des Erhöhens eines Abstands zwischen Heizeinheiten (S140) des Erhöhens eines Abstands zwischen jeder der Vielzahl oberer Heizeinheiten (410, 420), die angeordnet sind, dass sie jeweils der Vielzahl unterer Heizeinheiten (310, 320, 330) zugewandt sind, und jeder der Vielzahl unterer Heizeinheiten (310, 320, 330), bevor der einstufige Bewegungsschritt der Kammeranordnung (S150) durchgeführt wird, in einem Fall, in dem die Beibehaltungszeit in dem Heiz- und Messbetriebsstartschritt (S120) länger ist als eine voreingestellte Referenzbeibehaltungszeit (S130); und
einen Schritt des Verringerns eines Abstands zwischen Heizeinheiten (S160) des Verringerns des Abstands zwischen jeder der Vielzahl oberer Heizeinheiten (410, 420) und jeder der Vielzahl unterer Heizeinheiten (310, 320, 330), nachdem der einstufige Bewegungsschritt der Kammeranordnung (S150) durchgeführt wurde,
wobei in dem Schritt des Erhöhens des Abstands zwischen Heizeinheiten (S140) die Vielzahl oberer Heizeinheiten (410, 420) und die Vielzahl unterer Heizeinheiten (310, 320, 330) derartig ausgebildet sind, dass der Abstand zwischen jeder der Vielzahl oberer Heizeinheiten (410, 420) und jeder der Vielzahl unterer Heizeinheiten (310, 320, 330) größer ist als eine Höhe jeder der Vielzahl Kammern (210) und
wobei in dem Schritt des Verringerns eines Abstands zwischen Heizeinheiten (S160) der Abstand zwischen jeder der Vielzahl oberer Heizeinheiten (410, 420) und jeder der Vielzahl unterer Heizeinheiten (310, 320, 330) der Höhe jeder der Vielzahl Kammern (210) entspricht.

13. RT-PCR-Messverfahren nach Anspruch 11, wobei in dem einstufigen Bewegungsschritt der Kammeranordnung (S150) die Vielzahl Kammern (210) um einen voreingestellten Winkel um ein Rotationszentrum herum gedreht werden, das in der Basiseinheit (100) ausgebildet ist,
wobei die Vielzahl unterer Heizeinheiten (310, 320, 330) eine erste untere Heizeinheit (310), die bei einer ersten Temperatur (T₁) arbeitet, eine zweite untere Heizeinheit (320), die bei einer zweiten Temperatur (T₂) arbeitet, und eine dritte untere Heizeinheit (330) umfassen, die bei einer dritten Temperatur (T₃) arbeitet,
wobei die Vielzahl oberer Heizeinheiten (410, 420), die jeweils der Vielzahl unterer Heizeinheiten (310, 320, 330) zugewandt sind, eine erste obere Heizeinheit (410), die der ersten unteren Heizeinheit (310) zugewandt ist und bei der ersten Temperatur (T₁) arbeitet, eine zweite obere Heizeinheit (420), die der zweiten unteren Heizeinheit (320) zugewandt ist und bei der zweiten Temperatur (T₂) arbeitet, und eine dritte obere Heizeinheit umfassen, die der dritten unteren Heizeinheit (330) zugewandt ist und bei der dritten Temperatur (T₃) arbeitet, und
wobei die tragbare RT-PCR-Vorrichtung (1) die Vielzahl unterer Heizeinheiten (310, 320, 330) und die Vielzahl oberer Heizeinheiten (410, 420) derartig steuert, dass die Temperaturen aufrechterhalten werden, bei denen die Vielzahl unterer Heizeinheiten (310, 320, 330) bzw. die Vielzahl oberer Heizeinheiten (410, 420) arbeiten.

## Revendications

1. Dispositif de RT-PCR portable (1) comprenant :
une unité de base (100) dans laquelle est formé un espace de montage ;
une pluralité **d'unités** de chauffage inférieures (310, 320, 330) montées dans l'espace de montage dans l'unité de base (100) ;
une unité de mesure optique inférieure (500) montée dans l'unité de base (100) et disposée à une position différente de celles de la pluralité d'unités de chauffage inférieures (310, 320, 330) et délivrant de la lumière de mesure ou recevant la lumière de mesure ; et
un ensemble de chambres (200) comportant une pluralité de chambres (210) qui sont installées sur la pluralité d'unités de chauffage inférieures (310, 320, 330) et l'unité de mesure optique inférieure (500), respectivement, chaque chambre (210) étant disposée de manière à être mobile depuis une unité de la pluralité d'unités de chauffage inférieures (310, 320, 330) jusqu'à une autre unité de la pluralité d'unités de chauffage inférieures (310, 320, 330) ou jusqu'à l'unité de mesure optique inférieure (500),
dans lequel chaque chambre de la pluralité de chambres (210) comprend :
un corps de chambre (211) destiné à accueillir une unité de chambre (230) dans laquelle est formé un espace d'accueil d'unité d'échantillon (232) à l'intérieur duquel une unité d'échantillon (250) est accueillie,
**caractérisé en ce que** le corps de chambre (211) est formé pour recevoir l'unité de chambre (230) ayant un corps d'unité de chambre (231) dans lequel est formé l'espace d'accueil d'unité d'échantillon (232) pour accueillir l'unité d'échantillon (250) ayant un rapport de forme, c'est-à-dire un rapport hauteur/largeur, qui est supérieur à 0 et inférieur à 1 ; et une unité de bouchon (233) couvrant l'espace d'accueil d'unité d'échantillon (232) dans le corps d'unité de chambre (231) depuis le dessus, et
le dispositif de RT-PCR portable (1) comprenant en outre une unité de couvercle (600) disposée au-dessus de l'unité de base (100) et couvrant l'espace de montage ; une pluralité d'unités de chauffage supérieures (410, 420) dont chacune est disposée entre chaque unité de la pluralité d'unités de chauffage inférieures (310, 320, 330) et l'unité de couvercle (600) et qui sont sélectivement mises en contact avec des surfaces supérieures, respectivement, de la pluralité de chambres (210) ; et
une unité de mesure optique supérieure faisant face à l'unité de mesure optique inférieure (500), recevant la lumière de mesure émise depuis l'unité de mesure optique inférieure (500) ou délivrant la lumière de mesure vers l'unité de mesure optique inférieure (500) ; et
dans lequel la pluralité d'unités de chauffage supérieures (410, 420) et la pluralité d'unités de chauffage inférieures (310, 320, 330) sont formées de telle sorte qu'une distance entre chaque unité de la pluralité d'unités de chauffage supérieures (410, 420) et chaque unité de la pluralité d'unités de chauffage inférieures (310, 320, 330) est variable,
dans lequel, dans un cas où chaque chambre de la pluralité de chambres (210) est disposée entre chaque unité de la pluralité d'unités de chauffage supérieures (410, 420) et chaque unité de la pluralité d'unités de chauffage inférieures (310, 320, 330) et n'est pas déplacée pendant un temps de maintenance (Tₘ), la distance entre chaque unité de la pluralité d'unités de chauffage supérieures (410, 420) et chaque unité de la pluralité d'unités de chauffage inférieures (310, 320, 330) correspond à une hauteur de chaque chambre de la pluralité de chambres (210), et
dans lequel, dans un cas où le temps de maintenance (Tₘ) expire et où la pluralité de chambres (210) sont déplacées vers d'autres unités de chauffage supérieures (410, 420), respectivement, et vers **d'autres** unités de chauffage inférieures (310, 320, 330), respectivement, la distance entre chaque unité de la pluralité d'unités de chauffage supérieures (410, 420) et chaque unité de la pluralité d'unités de chauffage inférieures (310, 320, 330) est réglée pour être supérieure à la hauteur de chaque chambre de la pluralité de chambres (210).

2. Dispositif de RT-PCR portable (1) selon la revendication 1, dans lequel l'ensemble de chambres (200) comprend en outre :
une unité de déplacement de chambres (220) destinée à déplacer la pluralité de chambres (210),
dans lequel la pluralité de chambres (210) sont disposées autour d'un centre de rotation formé dans l'unité de base (100) de manière à être espacées d'une distance prédéfinie, et
dans lequel l'unité de déplacement de chambres (220) fait tourner la pluralité de chambres (210) autour du centre de rotation dans une direction en même temps.

3. Dispositif de RT-PCR portable (1) selon la revendication 2, dans lequel l'unité de déplacement de chambres (220) comprend :
une pluralité de supports d'accouplement (222), dont des premières parties d'extrémité sont accouplées à la pluralité de chambres (210), respectivement ; et
un axe de rotation (221) auquel des deuxièmes parties d'extrémité de la pluralité de supports d'accouplement (222) sont accouplées et qui est disposé de manière à être rotatif autour du centre de rotation,
dans lequel la rotation de l'unité de déplacement de chambres (220) est conçue pour être arrêtée pendant un temps de maintenance (Tₘ), et l'unité de déplacement de chambres (220) est conçue pour tourner pendant un temps de déplacement (Tᵣ), et
dans lequel le temps de maintenance (Tₘ) est réglé pour être plus long que le temps de déplacement (Tᵣ).

4. Dispositif de RT-PCR portable (1) selon la revendication 3, dans lequel une première unité de guidage (710) destinée à guider la rotation de chaque chambre de la pluralité de chambres (210) est formée pour être positionnée entre une unité de la pluralité d'unités de chauffage inférieures (310, 320, 330) et une autre unité de la pluralité d'unités de chauffage inférieures (310, 320, 330) ou l'unité de mesure optique inférieure (500),
dans lequel la première unité de guidage (710) est formée de manière à présenter une courbure correspondant à un rayon de courbure d'un cercle imaginaire qui est formé quand la pluralité de chambres (210) sont tournées, et
dans lequel une rainure de guidage ou une saillie de guidage qui est enclenchée avec la première unité de guidage (710) est formée dans ou sur une partie inférieure de chaque chambre de la pluralité de chambres (210).

5. Dispositif de RT-PCR portable (1) selon la revendication 4, dans lequel une deuxième unité de guidage (720) qui est accouplée à la première unité de guidage (710), présente le même rayon de courbure que la première unité de guidage (710), et est sélectivement enclenchée avec la rainure de guidage dans chaque chambre de la pluralité de chambres (210) ou la saillie de guidage sur celles-ci, est formée pour être positionnée sur des surfaces supérieures de l'unité de chauffage inférieure (310, 320, 330) et l'unité de mesure optique inférieure (500).

6. Dispositif de RT-PCR portable (1) selon la revendication 2, dans lequel l'unité de chauffage inférieure (310, 320, 330) comprend :
une première unité de chauffage inférieure (310) conçue pour fonctionner à une première température (T₁) ;
une deuxième unité de chauffage inférieure (320) conçue pour fonctionner à une deuxième température (T₂) ; et
une troisième unité de chauffage inférieure (330) conçue pour fonctionner à une troisième température (T₃), et
dans lequel la première unité de chauffage inférieure (310) et la troisième unité de chauffage inférieure (330) sont symétriques par rapport au centre de rotation, et
la deuxième unité de chauffage inférieure (320) et l'unité de mesure optique inférieure (500) sont symétriques par rapport au centre de rotation.

7. Dispositif de RT-PCR portable (1) selon la revendication 6, dans lequel la première température (T₁) est réglée pour être supérieure à la troisième température (T₃), et la troisième température (T₃) est réglée pour être supérieure à la deuxième température (T₂), et
dans lequel la première unité de chauffage inférieure (310), la deuxième unité de chauffage inférieure (320) et la troisième unité de chauffage inférieure (330) sont conçues pour être maintenues à leurs températures réglées respectives.

8. Dispositif de RT-PCR portable (1) selon la revendication 1, dans lequel chaque unité de la pluralité d'unités de chauffage inférieures (310, 320, 330) est formée sous la forme d'une plaque,
dans lequel des surfaces inférieures de la pluralité de chambres (210) sont mises en contact complet avec la pluralité d'unités de chauffage inférieures (310, 320, 330), respectivement,
dans lequel un espace d'insertion d'unité de chambre (215) à l'intérieur duquel l'unité de chambre (230) doit être insérée est formé dans le corps de chambre (211) de chaque chambre de la pluralité de chambres (210), et
dans lequel l'espace d'insertion d'unité de chambre (215) est formé de telle sorte qu'une largeur de ce dernier correspond à une largeur de chaque unité de la pluralité d'unités de chambre (230).

9. Dispositif de RT-PCR portable (1) selon la revendication 1, dans lequel une solution de mesure est accueillie dans l'espace d'accueil d'unité d'échantillon (232) dans chaque unité de la pluralité d'unités de chambre (230), et l'espace d'accueil d'unité d'échantillon (232) est formé de telle sorte que le rapport de forme de ce dernier est supérieur à 0 et inférieur à 1, et
dans lequel l'espace d'accueil d'unité d'échantillon (232) est formé de telle sorte qu'un volume de ce dernier va de 20 µl à 100 µl.

10. Dispositif de RT-PCR portable (1) selon la revendication 1, dans lequel l'unité d'échantillon (250) est formée avec une structure de membrane constituée d'un matériau poreux.

11. Procédé de mesure de RT-PCR qui utilise le dispositif de RT-PCR portable (1) de la revendication 1, le procédé comprenant :
une étape d'introduction des unités d'échantillon (S110) consistant à accueillir une pluralité d'unités de chambre (230), dans chacune desquelles l'unité d'échantillon (250) est accueillie, à l'intérieur de la pluralité de chambres (210), respectivement ;
une étape de lancement d'opération de chauffage et de mesure (S120) consistant à réaliser une opération de chauffage et de mesure sur l'unité d'échantillon (250) dans un état où l'unité d'échantillon (250) est introduite ;
une étape de déplacement par cran de l'ensemble de chambres (S150) consistant à déplacer la pluralité de chambres (210) d'un cran dans un cas où un temps de maintenance (Tₘ) dans l'étape de lancement d'opération de chauffage et de mesure (S120) est plus long qu'un temps de maintenance de référence prédéfini (S130) ; et
une étape de notification de résultat de mesure (S180) consistant à fournir une notification d'un résultat de mesure dans un cas où un cycle de mesure pour la pluralité de chambres (210) est réglé pour être plus long qu'un cycle de référence prédéfini (S170),
dans lequel un cycle de mesure est défini comme quatre crans desquels la pluralité de chambres (210) est déplacée.

12. Procédé de mesure de RT-PCR selon la revendication 11, comprenant en outre :
une étape d'accroissement de la distance entre unités de chauffage (S140) consistant à accroître une distance entre chaque unité de la pluralité d'unités de chauffage supérieures (410, 420) qui sont disposées pour faire face à la pluralité d'unités de chauffage inférieures (310, 320, 330), respectivement, et chaque unité de la pluralité d'unités de chauffage inférieures (310, 320, 330), avant que l'étape de déplacement par cran de l'ensemble de chambres (S150) soit réalisée, dans un cas où le temps de maintenance dans l'étape de lancement d'opération de chauffage et de mesure (S120) est plus long qu'un temps de maintenance de référence prédéfini (S130) ; et
une étape de réduction de la distance entre unités de chauffage (S160) consistant à réduire la distance entre chaque unité de la pluralité d'unités de chauffage supérieures (410, 420) et chaque unité de la pluralité d'unités de chauffage inférieures (310, 320, 330) après que l'étape de déplacement par cran de l'ensemble de chambres (S150) a été réalisée,
dans lequel, dans l'étape d'accroissement de la distance entre unités de chauffage (S140), la pluralité d'unités de chauffage supérieures (410, 420) et la pluralité d'unités de chauffage inférieures (310, 320, 330) sont formées de telle sorte que la distance entre chaque unité de la pluralité d'unités de chauffage supérieures (410, 420) et chaque unité de la pluralité d'unités de chauffage inférieures (310, 320, 330) est supérieure à une hauteur de chaque chambre de la pluralité de chambres (210), et
dans lequel, dans l'étape de réduction de la distance entre unités de chauffage (S160), la distance entre chaque unité de la pluralité d'unités de chauffage supérieures (410, 420) et chaque unité de la pluralité d'unités de chauffage inférieures (310, 320, 330) correspond à la hauteur de chaque chambre de la pluralité de chambres (210).

13. Procédé de mesure de RT-PCR selon la revendication 11 dans lequel, dans l'étape de déplacement par cran de l'ensemble de chambres (S150), la pluralité de chambres (210) sont tournées d'un angle prédéfini autour d'un centre de rotation formé dans l'unité de base (100),
dans lequel la pluralité d'unités de chauffage inférieures (310, 320, 330) incluent une première unité de chauffage inférieure (310) qui fonctionne à une première température (T₁), une deuxième unité de chauffage inférieure (320) qui fonctionne à une deuxième température (T₂), et une troisième unité de chauffage inférieure (330) qui fonctionne à une troisième température (T₃),
dans lequel la pluralité d'unités de chauffage supérieures (410, 420) qui font face à la pluralité d'unités de chauffage inférieures (310, 320, 330), respectivement, incluent une première unité de chauffage supérieure (410) qui fait face à la première unité de chauffage inférieure (310) et fonctionne à la première température (T₁), une deuxième unité de chauffage supérieure (420) qui fait face à la deuxième unité de chauffage inférieure (320) et fonctionne à la deuxième température (T₂), et une troisième unité de chauffage supérieure qui fait face à la troisième unité de chauffage inférieure (330) et fonctionne à la troisième température (T₃), et
dans lequel le dispositif de RT-PCR portable (1) contrôle la pluralité d'unités de chauffage inférieures (310, 320, 330) et la pluralité d'unités de chauffage supérieures (410, 420) de manière à maintenir les températures auxquelles la pluralité d'unités de chauffage inférieures (310, 320, 330) et la pluralité d'unités de chauffage supérieures (410, 420), respectivement, fonctionnent.
